## Europäisches Patentamt
## European Patent Office
## Office européen des brevets

(19)

(11) Publication number: **0 021 758**
**B1**

(12)

# EUROPEAN PATENT SPECIFICATION

(45) Date of publication of patent specification: **04.04.84**

(51) Int. Cl.³: **A 61 D 7/00**

(21) Application number: **80302011.4**

(22) Date of filing: **16.06.80**

(54) Veterinary preparations for ruminant animals.

(30) Priority: **27.06.79 GB 7922422**

(43) Date of publication of application:
**07.01.81 Bulletin 81/1**

(45) Publication of the grant of the patent:
**04.04.84 Bulletin 84/14**

(84) Designated Contracting States:
**BE CH DE FR GB LI NL**

(56) References cited:
EP - A - 0 010 987
FR - A - 2 393 576
GB - A - 1 264 731
GB - A - 1 471 465
US - A - 3 625 214
US - A - 3 710 795
US - A - 3 832 252
US - A - 3 896 819
US - A - 3 901 232
US - A - 3 903 880
US - A - 3 967 618

(73) Proprietor: **BEECHAM GROUP PLC**
**Beecham House Great West Road**
**Brentford Middlesex (GB)**

(72) Inventor: **Brewer, Malcolm David**
**59 Green Lane Northgate**
**Crawley Sussex (GB)**
Inventor: **Leiper, John William Guthrie**
**10 Gorse End**
**Horsham Sussex (GB)**

(74) Representative: **Cresswell, Thomas Anthony et al,**
**European Patent Attorney Beecham**
**Pharmaceuticals Great Burgh Yew Tree Bottom**
**Road**
**Epsom Surrey KT18 5XQ (GB)**

(56) References cited:
**US - A - 3 993 073**
**US - A - 4 016 251**
**US - A - 4 055 178**
**US - A - 4 069 307**

Courier Press, Leamington Spa, England.

Veterinary preparations for ruminant animals

This invention relates to veterinary preparations for ruminant animals.

Ruminant animals, particularly cattle and sheep, form an important group of animals which require periodic administration of veterinary medicines for the treatment and alleviation of various conditions. For example, it is often desirable to treat such animals, either therapeutically or prophylactically, with mineral or vitamin supplements, antibiotics, systemic insecticides, detergents for the relief of cattle bloat, and/or anthelmintics or other antiparasitic agents. The repeated administration of such veterinary medicines to animals at frequent time intervals is expensive and inconvenient. There is therefore much need for a dosing system to be devised which would efficiently supply the veterinary medicine during prolonged periods of time after administration of a single preparation.

British Patent No. 1318259 describes a number of devices for retaining slow release veterinary medicament formulations in the rumen over an extended period of time, thereby achieving the desired result. This prolonged retention in the rumen is obtained by the devices having a relatively narrow first configuration which allows the devices to be administered *per os* to the ruminant, and a relatively broad second configuration which the devices assume or are caused to assume in the rumen thereby hindering or preventing their passage out of the rumen. A typical example of such a device specifically described in the Patent is a plastic cylindrical capsule containing a detergent for the control of bloat in cattle. The capsule is 150 mm long and 30 mm wide (thereby allowing *per os* administration), and consists of two half-cylinders hinged along one edge. The hinges are made from rubber and are biased so that the two half-cylinders spring apart in the rumen and thus become too wide to pass out through the rumen or to be regurgitated through the oesophagus. Each half-cylinder contains a gel of ethyl cellulose containing the desired anti-bloat agent which is leached from the gel by the rumen fluids over an extended period of time. The hinges are constructed so that under the rumen conditions they pull away from the half-cylinders after effective release of the agent thereby facilitating regurgitation of the fragmented device.

FR—A—2,393,576 describes a device for the slow release of a water soluble medicament in the rumen environment. The device depends for its action on the leaching out of a water soluble medicament from an essentially water insoluble polymer matrix by the rumen liquors. The device is so designed as to allow it to be constrained in a first configuration suitable for oral administration to a ruminant animal and, on release of the constraint, may move relative to the first configuration to adopt a second configuration which is suitable for retaining the device in the rumen.

EP—A—0 010 987 describes a device which may also be constrained in a configuration suitable for oral administration and may adopt a second configuration in the rumen and thereby be retained in the rumen. The device is suitable for use with water insoluble medicaments which are released during the slow erosion of an erodable polymer matrix. This published application falls within the terms of Article 54(3) E.P.C.

We have now discovered a veterinary preparation which also achieves the desired result and has the advantage of being simple, cheap and easily manufactured. In this preparation the formulation is contained in a plastics envelope which is capable of being constrained around the formulation to allow *per os* administration of the preparation, and in the rumen of unfolding from its constrained administration position to effectively block any passage of the preparation out of the rumen. It should be noted that such a preparation is in no way described or suggested by British Patent No. 1 318 259, which is in the mean concerned with more complex devices.

Accordingly the present invention provides a veterinary preparation for ruminant animals, which preparation comprises a veterinary medicament contained in a solid or semi-solid matrix serving as a slow release formulation for the medicament, and a foraminous plastics envelope enclosing the formulation, which is capable of being constrained in a position which allows *per os* administration of the preparation and is, in the rumen, capable of moving relative to this position to retain the preparation therein.

Preferably, the envelope is constructed of plastics netting material. The formulation is suitably held loosely inside the envelope. The plastics envelope must be sufficiently flexible to be capable of being constrained for administration, and sufficiently resilient to unfold from this constrained position at least to an extent sufficient to effectively block passage of the preparation from the rumen. The envelope must also of course be non-toxic, and be stable to the rumen environment at least for the period of time during which release of the medicament contained therein is desired or possible. Thus examples of suitable materials from which the envelope may be made include semi-synthetic and synthetic plastics materials such as cellulose acetate, polyvinyl chloride, nylon, polyethylene, ethylene vinyl acetate copolymer and polyurethane, and synthetic rubbers such as silicone rubber polymers. Preferred materials include polyethylene.

Preferred embodiments of the invention will now be described by reference to the accompanying drawings, in which:

Fig. 1 is a perspective view of a veterinary preparation according to the invention, and

Fig. 2 is a perspective view of the preparation of Fig. 1 in its administration form.

Fig. 3 is a perspective view of an alternative veterinary preparation.

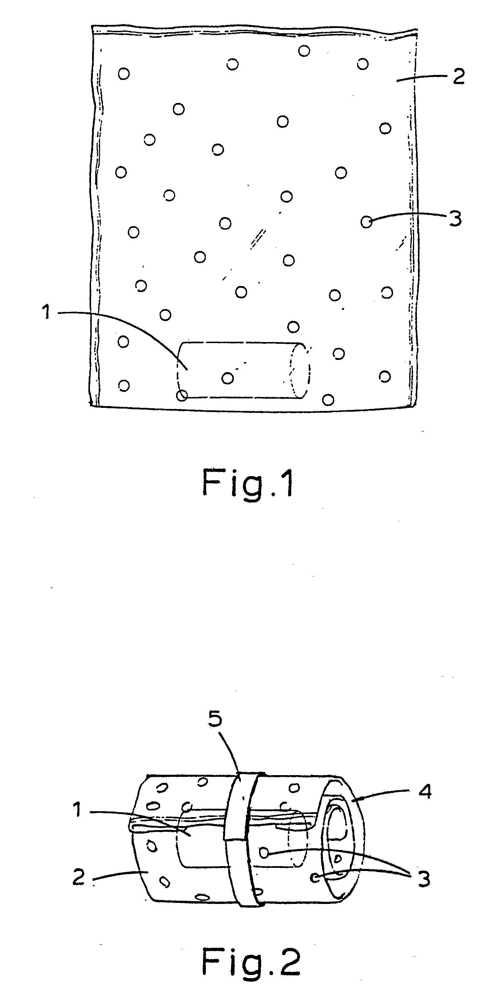

Referring to Figure 1, the slow release veterinary medicament formulation is shown in the form of cylindrical tablet 1 which is contained in a rectangular polyethylene envelope 2. The tablet 1 is shaped so that it fits neatly into one of the narrow ends of the envelope 2. The envelope is sealed at its edges, and is provided with a number of small holes 3 to render it permeable to rumen fluids so that, when the preparation is in the rumen, the tablet 1 is exposed to the rumen fluids and the medicament is leached therefrom at the desired slow rate.

For administration, the envelope 2 is folded around the tablet so that the preparation assumes an approximately cylindrical administration from 4, as shown in Fig. 2. The envelope is constrained temporarily in this position around the tablet 1 by a thin strip of paper 5 gummed with a water soluble adhesive affixed around its circumference. This strip of paper 5 is readily removed by the rumen fluids after administration of the preparation in its administration form 4, and the envelope 2 then unfolds due to its inherent resilience and in this way attains an effective cross-sectional area great enough to retain the preparation in the rumen. The unfolding of the envelope 2 from its constrained position is of course assisted by the natural movement of the rumen environment. In this unfolded form in the rumen, the tablet 1 enclosed within the envelope 2 is exposed to the rumen fluids through the holes 3 thereby allowing the medicament to be leached therefrom.

The size of the envelope 2 for administration to cattle is about 20 cm in length and about 15 cm in width, and for administration to sheep about 10 cm in length and about 7 cm in width. The cylindrical administration form of the preparation is about 5.5 cm in length and 2.5 cm in width for administration to cattle and about 5.0 cm in length and 2.0 cm in width for administration to sheep. This ensures easy and safe administration thereto. The polythene thickness is 0.1 mm, but a polythene thickness in the range 0.1 to 1 mm is preferably acceptable as this gives the required strength whilst retaining the flexibility necessary to allow the envelope to be constrained for administration as described.

The envelope described in the above embodiment is in effect a single container for the formulation, which formulation is free to move within the envelope when the envelope unfolds in the rumen. This envelope design has the advantage of simplicity, as the preparation may be formed simply by placing the formulation in a preformed open envelope and sealing its edges. A second formulation may also readily be incorporated in such an envelope. Other envelopes within the scope of the present invention include envelopes wherein the formulation is held within one part of the envelope by means of an additional seal or seals, envelopes in which two or more formulations are held separate from each other by a seal or seals, and envelopes wherein part of the envelope contains the formulation and the rest of the envelope is in the form of a flap or wing.

The envelope must be capable of being constrained in a position that allows *per os* administration of the preparation. It must also be capable of moving relative to this position once in the rumen to retain the preparation in the rumen. Thus normally the envelope will have a largest diameter in the range 4 to 30 cm, preferably 15 to 20 cm for cattle, and in the range 2 to 20 cm, preferably 7 to 12 cm for sheep. Suitably it is arranged into its administration position simply by folding it around the formulation enclosed therein.

In order for the preparation to be administered safely and easily to the ruminant animal, the administration form of the preparation must be of a size and shape that is easily swallowed. Obviously this size and shape will vary with the ruminant being dosed, but for example we found that for cattle a preparation in an approximately cylindrical shape of length about 4—6 cm and diameter about 2—3 cm is particularly suitable. Preferably these dimensions are about 5 and 2.5 cm respectively. Similarly for sheep, suitable cylindrical dimensions are length about 4—5 cm and diameter about 1—2 cm, preferably 4.5 cm and 1.5 cm respectively. The weight of the preparation will again depend on the ruminant being treated, and also on the medicament being used and the period of time during which this is to be made available within the rumen. Normally for cattle the weight is from about 10 to 200 g, and for sheep from about 2 to 40 g.

In order for the slow release formulation enclosed within the envelopes to be exposed to the rumen fluids when the envelope unfolds in the rumen the envelope must be permeable to these fluids. This requirement may readily be achieved by punching several small holes in the envelope as shown in Fig. 1. It has been found that if these holes are punched in a regular fashion in the envelope then there is a risk that lines formed by adjacent holes may act as points of weakness causing premature rupture of the envelope in the rumen with consequent loss of the formulation. As it is obviously essential that the envelope retains the formulation within its confines at least until the majority of medicament held in the formulation has been slowly leached therefrom, it is preferred that these holes be punched in a random manner in the envelope, as illustrated.

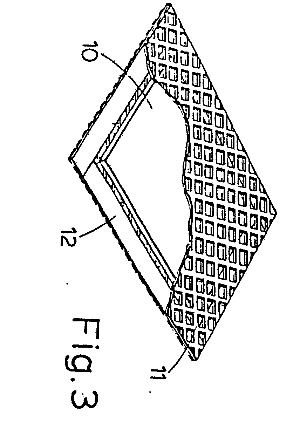

Referring to Fig. 3, the veterinary medicament formulation is in the form of a sheet 10 which is contained in a plastics netting envelope 11. The sheet is approximately 0.15 mm thick, 4 cm wide and 6 cm long, and the netting envelope is sealed along its edges by polyethylene strips 12 welded to the netting. The netting is suitably made from a low density polyethylene material "Netlon" (Registered Trade Mark) having regularly spaced holes about 3 mm×3 mm in size.

The device of Fig. 3 can be rolled up into a similar configuration as shown in Fig. 2 for administration *per os* to the sheep, and it will unroll to its planar configuration in the rumen.

For administration to the ruminant, the envelope is constrained in a position which will allow passage of the preparation down the animal's throat into its rumen. While this constraint may be applied to the envelope by the throat of the animal itself, it is normally preferred that some constraining means be associated with the envelope to hold it in this position for administration purposes. The constraining means is chosen so that it is quickly removed in the rumen environment to allow the envelope to unfold once it is in the rumen in the manner of the invention. This constraining means may be any device that is able to hold the envelope in its constrained position for administration, but is readily dissolved, destroyed, ruptured or otherwise removed by the rumen environment. Examples of suitable constraining means include gelatin string, gelatin tape, paper strips backed by water soluble adhesives, and water soluble paper.

The active medicament used in the slow release formulation may be one more mineral supplements, e.g. a cobalt or magnesium salt; vitamin supplements, especially those of the vitamin B class; systemic insecticides, e.g. Diazinon or Dichlorvos; detergents for the relief of cattle bloat; antibiotics, such as ampicillin, amoxycillin, cloxacillin, flucloxacillin and the like; and/or anthelmintics or other anti-parasitic compounds such as par bendazole, rafoxanide, or diamphenethide (Corriban) or thiabendazole, tetramisole, levamisole, morantel, pyrantel or oxybendazole for nematodes. Additional anthelmintics include fenbendazole, oxfendazole, albendazole, and avermectins, dihydroaver-mectins or combinations of these such as Iver-mectin. The invention is particularly applicable to the provision of anthelmintics for gastrointestinal nematodes and/or for use with anti-trypanosomiasis or anti-schistosomiasis compounds. It is also suitable for the administration of animal growth promotants.

The medicament is contained in a slow release formulation. Many such formulations which cause delay in the release of the active principle from the formulation have been described. For example, the active ingredient may merely be mixed with an inert diluent, and the mixture compounded so that release of the active ingredient from the matrix is regulated by the mechanical disintegration or slow dissolution of the formulation. For this purpose the active ingredient may be compounded with kaolin, calcium sulphate, chalk, carboxymethyl cellulose, ethyl cellulose, methyl cellulose, dextran starch, gelatine, dimethylsiloxane resins and the like. More sophisticated slow release formulations may also be utilised. For instance the active ingredient may be incorporated, by coacervation or precipitation techniques, in a polymer matrix or shell, from which it may be leached by the rumen fluids over an extended period of time.

The slow release formulation will be chosen so that the medicament contained therein is released in the rumen at the desired rate over the desired period of time.

The constrained preparation may conveniently be administered using a conventional dosing gun.

**Claims**

1. A veterinary preparation for ruminant animals, comprising a veterinary medicament contained in a solid or semisolid matrix serving as a slow release formulation for the medicament, and a carrier for the medicament which is capable of being constrained in a position which allows *per os* administration of the preparation and is, in the rumen, capable of moving relative to this position to retain the preparation therein, characterised in that the carrier comprises a foraminous plastics envelope (2) enclosing the formulation.

2. A veterinary preparation according to claim 1 characterised in that the envelope (2) is constructed of a synthetic polymer material.

3. A veterinary preparation according to claim 1 or claim 2, characterised in that the envelope is constrained by means of a constraining element which retains the envelope in a folded configuration and is removable by rumen fluids after administration.

4. A veterinary preparation according to claim 3 characterised in that the constraining element comprises gelatin or paper tape, strip, or string.

5. A veterinary preparation according to any one of the preceding claims, characterised in that the formulation is in sheet form.

6. A veterinary preparation according to any one of the preceding claims, characterised in that the medicament comprises an anthel-mintic.

7. A veterinary preparation according to any one of claims 1 to 5, characterised in that the medicament comprises an animal growth promotant.

8. A veterinary preparation according to any one of claims 1 to 5, characterised in that the medicament comprises a mineral additive or trace element additive.

9. A veterinary preparation according to any one of the preceding claims characterised in

7      **0 021 758**      8

that the matrix comprises an inert diluent which slowly disintegrates or dissolves in the rumen to release the medicament.

## Patentansprüche

1. Ein Veterinärpräparat für widerkäuende Tiere, welches ein Veterinärmedikament, enthalten in einer festen oder halbfesten Matrix, welche als ein Rezeptur für das langsame Freisetzen des Medikaments dient, und einen Träger für das Medikament enthält, der fähig ist, in eine Anordnung bzw. Lage eingezwängt zu werden, die eine per os-Verabreichung des Präparates ermöglicht, und der im Pansen fähig ist, sich relativ zu dieser Lage zu bewegen, um das darin enthaltene Präparat zurückzuhalten, dadurch gekennzeichnet, daß der Träger eine mit Löchern versehene Hülle (2) aus Kunststoff umfaßt, welche die Rezeptur einschließt.

2. Ein Veterinärpräparat gemäß Anspruch 1, dadurch gekennzeichnet, daß die Hülle (2) aus einem synthetischen Polymermaterial hergestellt ist.

3. Ein Veterinärpräparat gemäß Anspruch 1 oder 2, dadurch gekennzeichnet, daß die Hülle mittels eines zusammenzwängenden Elementes zusammengezwängt ist, welches die Hülle in einer gefalteten Anordnung hält und nach der Verabrechung durch die Pansenflüssigkeiten entfernbar ist.

4. Ein Veterinärpräparat gemäß Anspruch 3, dadurch gekennzeichnet, daß das zusammenzwängende Element ein Band, einen Streifen oder eine Schnur aus Gelatine oder aus Papier enthält.

5. Ein Veterinärpräparat gemäß irgendeinem der vorhergehenden Ansprüche, dadurch gekennzeichnet, daß die Formulierung in Blattform vorliegt.

6. Ein Veterinärpräparat gemäß irgendeinem der vorhergehenden Ansprüche, dadurch gekennzeichnet, daß das Medikament ein Wurmmittel enthält.

7. Ein Veterinärpräparat gemäß irgendeinem der Ansprüche 1 bis 5, dadurch gekennzeichnet, daß das Medikament einen Promotor für das Wachstum eines Tieres enthält.

8. Ein Veterinärpräparat gemäß irgendeinem der Ansprüche 1 bis 5, dadurch gekennzeichnet, daß das Medikament einen Mineralzusatzstoff oder einen Spurenelement-Zusatzstoff enthält.

9. Ein Veterinärpräparat gemäß irgendeinem der vorhergehenden Ansprüche, dadurch gekennzeichnet, daß die Matrix ein inertes Verdünnungsmittel enthält, welches sich im Pansen langsam zersetzt oder auflöst und das Medikament freisetzt.

## Revendications

1. Préparation vétérinaire pour ruminants, renfermant un médicament à usage vétérinaire contenu dans une matrice solide ou semi-solide, servant de composition ou formule à libération lente pour le médicament, et un support ou véhicule pour le médicament, capable d'être maintenu dans une condition permettant l'administration par voie orale de la préparation et qui, dans la panse, est capable de se déplacer par rapport à cette condition pour retenir la préparation dans la panse, caractérisée en ce que le support est constitué par une enveloppe perforée en matière plastique (2) qui entoure la composition.

2. Préparation vétérinaire suivant la revendication 1, caractérisée en ce que l'enveloppe (2) est réalisée en une matière polymère synthétique.

3. Préparation vétérinaire suivant la revendication 1 ou 2, caractérisée en ce que l'enveloppe est maintenue au moyen d'un élément de retenue, qui maintient cette enveloppe selon une configuration repliée et qui peut être enlevé ou éliminé par les fluides de la panse après administration.

4. Préparation vétérinaire suivant la revendication 3, caractérisée en ce que l'élément de retenue est constitué par un ruban, une bande ou un cordon de gélatine ou de papier.

5. Préparation vétérinaire suivant l'une quelconque des revendications précédentes, caractérisée en ce que la composition se présente sous un aspect foliiforme.

6. Préparation vétérinaire suivant l'une quelconque des revendications précédentes, caractérisée en ce que le médicament est constitué par un anthelminthique.

7. Préparation vétérinaire suivant l'une quelconque des revendications 1 à 5, caractérisée en ce que le médicament est constitué par un agent favorisant ou activant la croissance de l'animal.

8. Préparation vétérinaire suivant l'une quelconque des revendications 1 à 5, caractérisée en ce que le médicament est constitué par un additif minéral ou par un additif formé d'oligo-éléments.

9. Préparation vétérinaire suivant l'une quelconque des revendications précédentes, caractérisée en ce que la matrice est constituée par un diluant inerte qui se désintègre ou se dissout lentement dans la panse pour libérer le médicament.

5

Fig.1

Fig.2

Fig.3